# EUROPEAN PATENT SPECIFICATION

(11) **EP 1 287 793 B1**
(45) Date of publication and mention of the grant of the patent: **12.01.2005**
(21) Application number: 02255860.5
(22) Date of filing: 22.08.2002
(51) Int. Cl.: A61F 2/36

(54) **Metallic prosthetic femoral component**
Femorale Prothesenkomponente aus Metall
Elément métallique d'une prothèse femorale

(30) Priority: 28.08.2001 GB 0120836
(43) Date of publication of application: 05.03.2003
(73) Proprietor: BENOIST GIRARD SAS, 14201 Hérouville-Saint-Clair Cédex (FR)
(72) Inventor: Raugel, Patrick, 37530 Charge (FR); Storer, John Andrew, 80801 München (DE)
(74) Representative: Bridge-Butler, Alan James

(56) References cited:
- EP-A- 0 106 946
- EP-A- 0 124 443
- DE-A- 3 907 530
- FR-A- 2 655 847
- GB-A- 1 371 335
- US-A- 4 687 488
- US-A- 4 718 914
- US-A- 5 549 703

## Description

This invention relates to a metallic prosthetic femoral component which is not only applicable for use with a natural acetabular socket, but can also be used with prosthetic acetabular sockets.

Natural acetabular sockets require a large ball head and these, when made of metal, tend to be heavy, especially in comparison with smaller diameter ball heads which are used with artificial sockets. To reduce weight it is known to make them hollow.

The present invention provides a construction in which the weight of the ball head and its stem can be reduced and allows the use of small femoral stem due to the greater strength of the construction.

DE 39 07 530 shows a method of constructing a prosthetic femoral component by pressing a hollow ball shaped head and welding it to a neck or collar, which can be subsequently connected to a femoral stem, for example by using an engaging tapered spigot. This type of construction provides no advantages over well known constructions of femoral components apart from a reduction in the weight of the ball and is unsuitable for use with large diameter balls which, because of their size, require a more stable form of attachment to the stem.

According to the present invention a metallic prosthetic femoral component comprises a hollow femoral ball head connected to a forged femoral stem characterised in that said stem has a short disc-like spigot which has an outer wall which is secured by welding in an open mouth in said hollow ball head.

The invention provides a solution to reduce the weight of the implant by increasing the strength of the material and providing a joint between the stem and the ball of larger diameter which has a larger welding line and thus greater strength. Thus for a similar strength the mass of metal necessary can be reduced.

By using a forged femoral stem the implant has greater strength than the cast stems which are normally employed for equivalent shape and size of stem and as a result the implant is less bulky and can fit on a wider range of femur.

Preferably the spigot is cup shaped to provide an inner cavity. This enables the stem not only to be stronger but it is also lighter.

The hollow head can be cast but preferably it is also forged so that the improved strength of the material allows it to have a thinner load bearing wall, or the hollow head can be turned from a bar of solid material to achieve the same result.

The result of using forged components enables the invention to be applied not only to large stems but also to small ones which can be used in smaller femurs.

The hollow ball member can have an interior which is substantially part-spherical or any other convenient shape with a substantially cylindrical mouth portion into which the spigot is secured.

The invention also includes a method of forming a prosthetic femoral component having a stem and a hollow femoral ball head characterised by forging a stem having a distal shaft portion and a proximal integrally forged short disc-like spigot having an outer wall; forming a hollow head having an open mouth for receiving said spigot, said mouth circumference dimensioned to be a close fit around said outer wall; and welding said hollow head to said spigot around the circumference of said mouth and said outer wall.

The invention can be performed in various ways but one embodiment will now be described by way of example and with reference to the accompanying drawings in which :
Figure 1 is a side elevation of a metallic prosthetic femoral component according to the invention;
Figure 2 is an isometric view of the component shown in Figure 1;
Figure 3 is a front elevation of a femoral stem for use in the invention;
Figure 4 is a side elevation of the stem shown in Figure 3;
Figure 5 is a part cross-sectional side elevation viewed in the direction of the line A of Figure 3;
Figure 6 is a cross-sectional view of the stem on the line VI-VI of Figure 3;
Figure 7 is a cross-sectional view on the line VII-VII of Figure 3;
Figure 8 is a cross-sectional view on the line VIII-VIII of Figure 3;
Figure 9 is a front elevation of a hollow ball head for use in the invention;
Figure 10 is a cross-sectional view of the ball head shown in Figure 9; and,
Figure 11 is an isometric view of the ball head shown in Figures 9 and 10.

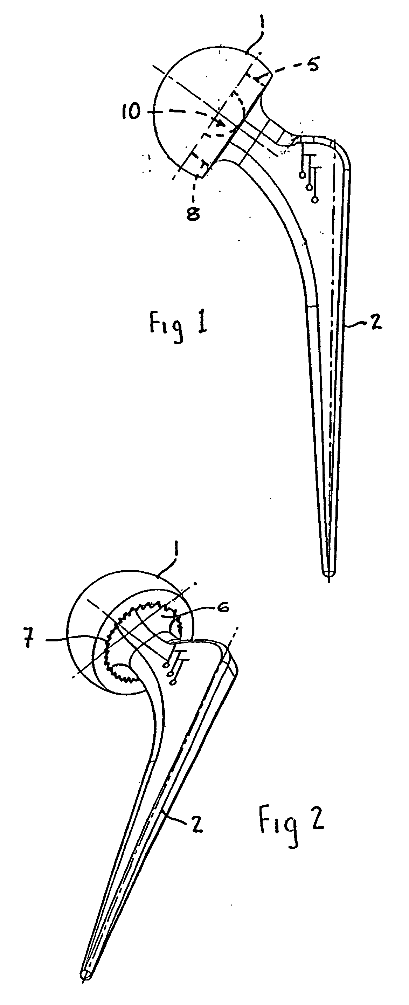

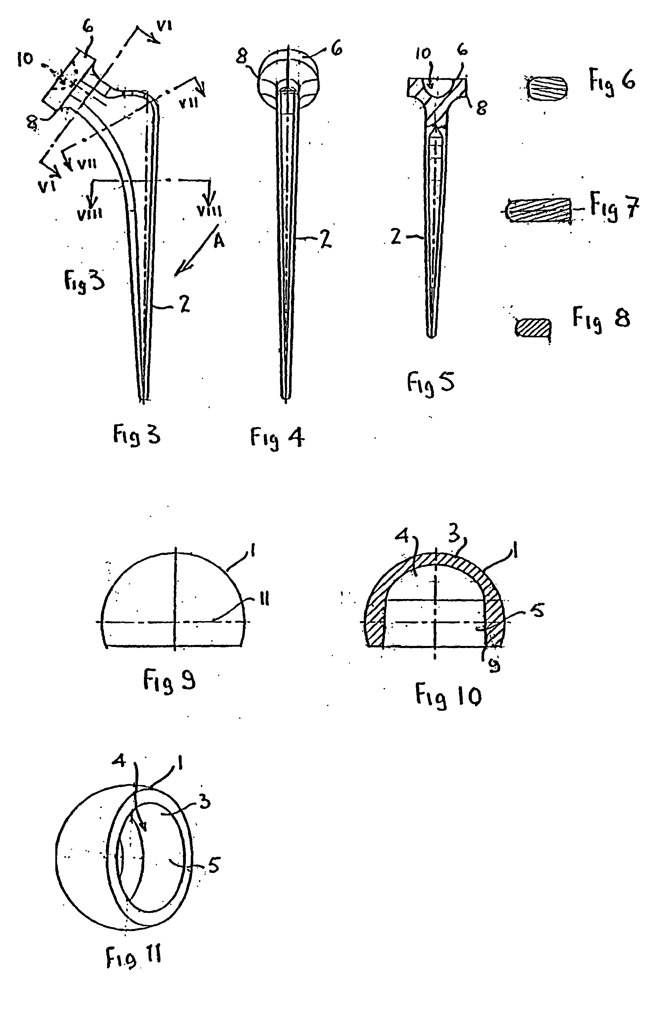

As shown in Figures 1 to 11 a metallic prosthetic femoral component comprises a femoral ball head 1 and a femoral stem 2. The ball head 1 has a load carrying wall 3, most clearly shown in figures 10 and 11, and a hollow interior 4.

The hollow interior 4 is substantially part-spherical or any other convenient shape, for example a flat bottomed cup shape, with a substantially cylindrical mouth portion 5, the shape can be dictated by the strength of the thinnest wall and the manufacturing process. The stem 2 has a short disc-like spigot 6 the outer wall 8 of which is dimensioned to be a close fit in the mouth 5 of the ball head 1 to which it is joined by connection means in the form of welding indicated by reference numeral 7 on Figure 2. If desired the distal circumference of the outer wall 8 of the spigot 6 and the outer circumference 9 of the mouth portion 5 of the ball head 1 can be bevelled to provide a greater welding surface area.

The spigot 6 is cup-shaped to provide a cavity 10.

The spigot 6 closes the mouth 5 and leaves the major portion of the interior of the head 1 hollow.

The stem 2 is forged and due to the use of this process, together with the particular shape of the spigot which is produced by using a cup shape with a cavity, a particularly strong construction is achieved. It will be appreciated that when the head is forged the metal can flow around the former which produces the cavity 10 to form a very strong shape which is also lighter for its size than a solid casing. It thus improves the strength of the outer ring of metal which provides the spigot and to which the hollow head is welded.

Further reductions in weight can also be achieved by using a forged hollow head, this allows the thickness of the load carrying wall 3 to be reduced to provide a lighter construction.

Due to the additional strength of the spigot on the stem this construction can be used with small sized implants which are normally not sufficiently reliable and cannot provide sufficient strength due to the use of castings.

It has been found however that a stem of the type described can also be successfully used with cast ball heads and is satisfactory due to its inherent strength.

The ball head and stem can be made from any convenient material, for example stainless steel.

In the construction shown in Figures 3 to 11 of the drawings the outer peripheral wall of the spigot 6 is substantially parallel, as are the walls of the mouth 5 but, if desired, they could be tapered to provide a tapered fit prior to welding as shown in Figure 1.

## Claims

1. A metallic prosthetic femoral component comprising a hollow femoral ball head (1) connected to a forged femoral stem (2) **characterised in that** said stem (2) has a short disc-like spigot (6) which has an outer wall (8) which is secured by welding in an open mouth (5) in said hollow ball head (1).

2. A metallic prosthetic femoral component as claimed in claim 1 **characterised in that** said spigot (6) is cup shaped to provide an inner cavity (10).

3. A metallic prosthetic femoral component as claimed in claim 1 or claim 2 **characterised in that** the ball head (1) is forged or is turned from a bar or solid material.

4. A metallic prosthetic femoral component as claimed in any one of preceding claims 1, to 3 **characterised in that** the ball head (1) has an interior which is substantially part-spherical with a substantially cylindrical mouth portion (5) into which the spigot (6) is secured.

5. A metallic prosthetic femoral component as claimed in claim 4 **characterised in that** the outer peripheral wall (8) of the spigot (6) is tapered, as are the walls of the mouth portion (5) to provide a taper fit

6. A method for forming a prosthetic femoral component having a femoral stem (2) and a hollow femoral ball head (1) **characterised by**:
forging a stem (2) having a distal shaft portion and a proximal integrally forged short disc-like spigot (6) having an outer wall (8);
forming a hollow head (1) having an open mouth (5) for receiving said spigot (6), said mouth (5) circumference dimensioned to be a close fit around said outer wall (8); and
welding said hollow head (1) to said spigot (6) around the circumference of said mouth (5) and said outer wall (8).

7. The method as set forth in claim 6 **characterised in that** said spigot is cup-shaped to provide an inner cavity (10).

8. The method as set forth in claim 6 or claim 7 **characterised in that** said head is forged or is formed from a bar of solid material.

9. The method as set forth in any one of preceding claims 6, 7 or 8 **characterised in that** the ball head (1) has an interior which is substantially part-spherical with a . substantially cylindrical mouth portion (5) into which the spigot (6) is secured.

10. The method as set forth in any one of preceding claims 6 to 9 **characterised in that** the outer peripheral wall (8) of the spigot (6) is tapered, as are the walls of the mouth portion (5) to provide a taper fit.

## Patentansprüche

1. Metallische prothetische femorale Komponente, umfassend einen mit einem geschmiedeten femoralen Schaft (2) verbundenen hohlen femoralen Kugelkopf (1), **dadurch gekennzeichnet, dass** der Schaft (2) einen kurzen scheibenartigen Zapfen (6) aufweist, der eine Außenwand (8) besitzt, die durch Schweißen in einer offenen Mundöffnung (5) im hohlen Kugelkopf (1) befestigt ist.

2. Metallische prothetische femorale Komponente nach Anspruch 1, **dadurch gekennzeichnet, dass** der Zapfen (6) napfförmig ist, um einen inneren Hohlraum (10) bereit zu stellen.

3. Metallische prothetische femorale Komponente nach Anspruch 1 oder Anspruch 2, **dadurch gekennzeichnet, dass** der Kugelkopf (1) geschmiedet ist oder aus einer Stange oder massivem Material gedreht ist.

4. Metallische prothetische femorale Komponente nach einem der vorangehenden Ansprüche 1 bis 3, **dadurch gekennzeichnet, dass** der Kugelkopf (1) ein Inneres aufweist, das im Wesentlichen teilsphärisch ist, mit einem im Wesentlichen zylindrischen Mundöffnungsteil (5), in dem der Zapfen (6) befestigt ist.

5. Metallische prothetische femorale Komponente nach Anspruch 4, **dadurch gekennzeichnet, dass** die äußere Umfangswand (8) des Zapfens (6) ebenso wie die Wände des Mundöffnungsteils (5) konisch ist, um eine Kegelpassung zu liefern.

6. Verfahren zum Formen einer prothetischen femoralen Komponente mit einem femoralen Schaft (2) und einem hohlen femoralen Kugelkopf (1), **gekennzeichnet durch**:
Schmieden eines Schaftes (2) mit einem distalen Schaftteil und einem proximalen integriert geschmiedeten kurzen scheibenartigen Zapfen (6), der eine Außenwand (8) besitzt;
Formen eines hohlen Kopfes (1) mit einer offenen Mundöffnung (5) zum Aufnehmen des Zapfens (6), wobei der Umfang der Mundöffnung (5) bemessen ist, um eine enge Passung um die Außenwand (8) herum zu bilden; und
Verschweißen des hohlen Kopfes (1) mit dem Zapfen (6) um den Umfang der Mundöffnung (5) und der Außenwand (8) herum.

7. Verfahren nach Anspruch 6, **dadurch gekennzeichnet, dass** der Zapfen napfförmig ist, um einen inneren Hohlraum (10) bereit zu stellen.

8. Verfahren nach Anspruch 6 oder Anspruch 7, **dadurch gekennzeichnet, dass** der Kopf geschmiedet wird oder aus einer Stange aus massivem Material geformt wird.

9. Verfahren nach einem der vorangehenden Ansprüche 6, 7 oder 8, **dadurch gekennzeichnet, dass** der Kugelkopf (1) ein Inneres aufweist, das im Wesentlichen teilsphärisch ist, mit einem im Wesentlichen zylindrischen Mundöffnungsteil (5), in dem der Zapfen (6) befestigt wird.

10. Verfahren nach einem der vorangehenden Ansprüche 6 bis 9, **dadurch gekennzeichnet, dass** die äußere Umfangswand (8) des Zapfens (6) ebenso wie die Wände des Mundöffnungsteils (5) konisch ist, um eine Kegelpassung zu liefern.

## Revendications

1. Composant fémoral prothétique métallique comprenant un pivot sphérique fémoral creux (1) relié à une tige fémorale forgée (2), **caractérisé en ce que** ladite tige (2) comporte un raccord en forme de disque court (6) qui comporte une paroi extérieure (8) qui est fixée par soudage dans une embouchure ouverte (5) dans ledit pivot sphérique creux (1).

2. Composant fémoral prothétique métallique selon la revendication 1, **caractérisé en ce que** ledit raccord (6) est en forme de coupelle, de façon à présenter une cavité intérieure (10).

3. Composant fémoral prothétique métallique selon la revendication 1 ou la revendication 2, **caractérisé en ce que** le pivot sphérique (1) est forgé ou est tourné à partir d'une barre ou d'un matériau plein.

4. Composant fémoral prothétique métallique selon l'une quelconque des revendications 1 à 3 qui précèdent, **caractérisé en ce que** le pivot sphérique (1) comporte un intérieur qui est sensiblement partiellement sphérique, avec une partie d'embouchure sensiblement cylindrique (5) dans laquelle est fixé le raccord (6).

5. Composant fémoral prothétique selon la revendication 4, **caractérisé en ce que** la paroi périphérique extérieure (8) du raccord (6) est effilée, tout comme les parois de la partie d'embouchure (5), de façon à réaliser une fixation conique.

6. Procédé pour former un composant fémoral prothétique comportant une tige fémorale (2) et un pivot sphérique fémoral creux (1), **caractérisé par** les étapes consistant à :
forger une tige (2) comportant une partie d'arbre distale et un raccord en forme de disque court forgé d'une seule pièce proximal (6) comportant une paroi extérieure (8) ;
former une tête creuse (1) comportant une embouchure ouverte (5) pour recevoir ledit raccord (6), la circonférence de ladite embouchure (5) étant dimensionnée de façon à produire une adaptation étroite autour de ladite paroi extérieure (8) ; et
souder ladite tête creuse (1) audit raccord (6) autour de la circonférence de ladite embouchure (5) et de ladite paroi extérieure (8).

7. Procédé selon la revendication 6, **caractérisé en ce que** ledit raccord est en forme de coupelle, de façon à présenter une cavité intérieure (10).

8. Procédé selon la revendication 6 ou la revendication 7, **caractérisé en ce que** ladite tête est forgée ou est formée à partir d'une barre de matériau plein.

9. Procédé selon l'une quelconque des revendications 6, 7 ou 8 qui précèdent, **caractérisé en ce que** le pivot sphérique (1) comporte un intérieur qui est sensiblement partiellement sphérique, avec une partie d'embouchure sensiblement cylindrique (5) dans laquelle est fixé le raccord (6).

10. Procédé selon l'une quelconque des revendications 6 à 9 qui précèdent, **caractérisé en ce que** la paroi périphérique extérieure (8) du raccord (6) est effilée, tout comme les parois de la partie d'embouchure (5), de façon à réaliser une fixation conique.
